(19) **Europäisches Patentamt**

**European Patent Office**

**Office européen des brevets**

(11) **EP 1 712 230 A1**

(12) **EUROPEAN PATENT APPLICATION**
published in accordance with Art. 158(3) EPC

(43) Date of publication:
**18.10.2006 Bulletin 2006/42**

(51) Int Cl.:
*A61K 31/438* (2006.01)  *A61P 1/02* (2006.01)
*A61P 27/02* (2006.01)  *A61P 43/00* (2006.01)
*C07D 491/107* (2006.01)

(21) Application number: **05790491.4**

(22) Date of filing: **04.10.2005**

(86) International application number:
**PCT/JP2005/018310**

(87) International publication number:
**WO 2006/038596 (13.04.2006 Gazette 2006/15)**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(30) Priority: **05.10.2004 JP 2004292611**

(71) Applicant: **Astellas Pharma Inc.**
**Tokyo 103-8411 (JP)**

(72) Inventors:
• **OKADA, Yohei**
**c/o Astellas Pharma Inc.**
**Chuo-ku Tokyo, 1038411 (JP)**

• **IKEDA, Ken**
**c/o Astellas Pharma Inc.**
**Chuo-ku Tokyo, 1038411 (JP)**
• **WANIBUCHI, Fumikazu**
**c/o Astellas Pharma Inc., 3-11**
**Chuo-ku Tokyo, 103841 (JP)**
• **YOSHIHARA, Keiichi**
**c/o Astellas Pharma Inc., 3-11,**
**Chuo-ku Tokyo, 1038411 (JP)**
• **KONDO, Hiromu**
**2-chome, Chuo-ku Tokyo; 1038411 (JP)**
• **KOJIMA, Hiroyuki**
**2-chome, Chuo-ku Tokyo; 1038411 (JP)**

(74) Representative: **Bates, Philip Ian**
**Reddie & Grose**
**16 Theobalds Road**
**London WC1X 8PL (GB)**

(54) **PHARMACEUTICAL COMPOSITION FOR XEROPHTHALMIA AND XEROSTOMIA TREATMENT**

(57) [Problem]
An agent for treating eye and mouth dryness, which does not accompanies undesirable actions is provided.
[Means for Resolution]
Administration of the compound A made it possible to accelerate tear and salivary fluid secretion without accompanying sweating and its sustained release administration enabled acceleration of lacrimal gland and salivary gland cell growth as well.

EP 1 712 230 A1

**Description**

[Technical Field]

This invention relates to a pharmaceutical composition for treatment of tear and salivary fluid drying, which comprises (-)-(S)-2,8-dimethyl-3-methylene-1-oxa-8-azaspiro[4.5]decane (to be referred to as compound A hereinafter) or a pharmaceutically acceptable salt thereof as the active ingredient. The invention also relates to a pharmaceutical composition for selective tear and salivary fluid secretion acceleration, which comprises the compound A or a pharmaceutically acceptable salt thereof as the active ingredient. The invention further relates to the use of the compound A or a pharmaceutically acceptable salt thereof for producing a sustained release pharmaceutical composition for tear and salivary fluid secretion acceleration. In addition, the invention relates to the pharmaceutical composition for treatment tear and salivary fluid dryness described in the above, which has a form of a sustained release preparation.

[Background of the Invention]

The mouth and eyes are one of the regions of the body most frequently exposed to the external environment. In general, it is known that saliva in human is carrying out important functions for the digestion of food, lubrication and protection of oral and digestive tract mucous membranes and infection protection, therefore insufficient secretion of saliva causes problems regarding oral hygiene and health. For example, these are unpleasant feeling inside the mouth, crevices in the oral mucous membrane and tongue, sleeplessness due to the unpleasant feeling, increase of periodontal disease and carious tooth due to breakdown of cleaning action and infection protective mechanism, dyspepsia, accumulation of food inside the mouth, dental plaque, extreme bad breath and the like ([Non-patent Reference 1], [Non-patent Reference 2] and [Non-patent Reference 3]).
In the meanwhile, tear fluid is carrying out an important role in maintaining normal visual function. That is, tear fluid is essential for the keeping of corneal refraction and refractive power, protection of cornea and conjunctiva, lubrication at the time of blink motion and infection protection by the secretion of lysozyme and IgA. It has been reported that reduction of tear fluid secretion causes ocular feeling of dryness, foreign body sensation, fatigue, itching or burning, and continuation of this phenomenon is apt to cause disorder of corneal epithelium and infection with fungi, bacteria and virus ([Non-patent Reference 1], [Non-patent Reference 4] and [Non-patent Reference 5]).
Though the cause of reduction of the secretion of saliva and tear fluid is varied, mainly rheumatic and autoimmune diseases such as rheumatoid arthritis, Sjogren syndrome and systemic lupus erythematosus, medical diseases such as diabetes mellitus, hepatic cirrhosis and renal failure, allergic keratoconjunctivitis, viral diseases such as AIDS, salivary gland and lacrimal gland damage associated with radiation therapy for cancer, aging, psychological fatigue and the like have been reported ([Non-patent Reference 6]).
Since the regeneration rate of salivary gland and lacrimal gland is very mild in general, it is considerably difficult to restore tissues destroyed or contracted by various causes ([Non-patent Reference 7]). In addition, dry mouth and dry eye have also been reported as side effects of the administration of various drugs including anti-hypertensive drug, antidepressant, antispasmodic agent, diuretic, muscle relaxant, anti-psychotic drug, anorectic and antiparkinsonism drug ([Non-patent Reference 1]).
Sjogren syndrome is a disease which shows dryness of eye, mouth and the like due to the occurrence of an exocrine gland hypofunction caused by an autoimmune reaction, and is set in rheumatic diseases including rheumatoid arthritis. Since the exocrine gland which underwent a disorder is replaced with connective tissues or inflammatory cells, the dryness continues so that the symptoms are irreversible ([Non-patent Reference 8]).
In the treatment of head and neck cancer, the exocrine gland is easily destroyed by a radiation irradiation of about 50 gray (Gy) to the head or neck, so that the secretion quantity of saliva and tear fluid is reduced. In addition, there are reports stating that the eye and mouth dryness starts just after the radiation irradiation and is progressive, persistent and not recoverable ([Non-patent Reference 9] and [Non-patent Reference 10])
When dry mouth and dry eye are generated, as described in the foregoing, one has to feel markedly many and serious pains persistently in daily life. Thus, proper countermeasure for them is strongly in demand.
The current dryness treatment is limited to symptomatic therapy as described below ([Non-patent Reference 1] and [Non-patent Reference 11]). Dry eye responds to the application of an eye drop of artificial tear fluid or autologous serum, but frequent use of these eye drops is needed. Though soft contact lenses are recommended for protecting cornea, a risk of causing infection is increased. A moisture chamber goggle is also used for preventing evaporation of tear fluid. Though lacrimal duct blocking by a punctal plug is also carried out for the purpose of preventing outflow of tear fluid from conjunctival sac into nasal cavity, this is very troublesome because application of artificial tear is essential and application of antibiotic eye drop is also essential.
In the meanwhile, the use of artificial saliva, gargles and buccal ointments has been attempted as a dry mouth countermeasure. However, such preparations are irritating or bitter or may have a problem such as short duration of moist action, so that their improvement is in demand. In addition, taking of liquids or hard candy is also carried out for treatment of mild dry mouth patients. However, since dry mouth patients are apt to undergo carious tooth and periodontal disease, the amount of sugar contained in the conventional candies and the like may have a problem. In addition, the liquid or hard candy is not effective for severe dry mouth in most cases, and they does not provide long-lasting relief even in the mild cases, so that further countermeasure is in demand.

By the way, it is known that muscarinic receptors are present on the lacrimal gland and salivary gland, and secretion of tear fluid and saliva occurs when this receptors are stimulated ([Non-patent Reference 12]). A muscarinic receptor agonist, pilocarpine or cevimeline, is already used for the treatment of dry eye or mouth. In addition, it is known that, during the radiation therapy of a head or neck cancer, the saliva secretion ability can be preserved by inhibiting radiation damage of the salivary gland through the prophylactic administration of these muscarinic receptor agonists ([Non-patent Reference 13] and [Non-patent Reference 14]). However, since the muscarinic receptors are present in many tissues in the body, such a treatment accompanies undesirable actions typified by sweating.

On the other hand, though it is known that the muscarinic receptor agonist accelerates cell growth of salivary gland, it is the present situation that its dosage necessary for the purpose is high far exceeding the saliva secretion acceleration dose ([Non-patent Reference 15]), and even a saliva secretion agent having a function to repair destroyed salivary gland is not resulting in its practical use in human.

The compound A is a muscarinic receptor agonist disclosed in [Patent Reference 1], [Patent Reference 2] and [Patent Reference 3].

Though the [Patent Reference 3].already reports on the possibility of applying the compound A to the treatment of dry eye, a "tear fluid and saliva secretion acceleration action" and a "glandular cell growth acceleration action" are not known.

In addition, it has been reported that the response of salivation is more marked than the benefit in ocular symptoms by a certain cause in treating dryness of Sjogren syndrome with pilocarpine ([Non-patent Reference 12]). Thus, it is considered that sufficient muscarinic receptor stimulation in lacrimal gland for the treatment of dry eye is attained at much higher dosage of a muscarinic receptor agonist than that for the treatment of dry mouth, which poses a problem of generating undesirable side effects with a high frequency. There is a demand for a pharmaceutical composition for the treatment of tear and salivary fluid drying which does not accompany undesirable actions typified by sweating, for the treatment of diseases that show both of the dry mouth and dry eye, such as Sjogren syndrome.

[Patent Reference 1].JP-B-5-44948

[Patent Reference 2].International Publication 92/20683

[Patent Reference 3].JP-A-2003-63964

[Non-patent Reference 1] Porter SR et al., "An update of the etiology and management of xerostomia", Oral Surgery Oral Medicine Oral Pathology, 2004, 97, pp. 28 - 46

[Non-patent Reference 2] Cassolato SF et al., "Xerostomia: Clinical Aspects and Treatment", Gerodontology, 2003, 20, pp. 64 - 77

[Non-patent Reference 3] Guggenheimer iJ et al., "Xerostomia, Etiology, Recognition and Treatment", Journal of American Dental Association, 2003, 134, pp. 61 - 69

[Non-patent Reference 4] Schaumberg DA et al., "Epidemiology of Dry Eye Syndrome", Advances in Experimental Medicine and Biology, 2002, 506, Pt B, pp. 989 - 998

[Non-patent Reference 5] Toda I et al., "Ocular Fatigue is the Major Symptom of Dry Eye", Acta Ophthalmologica, 1993, 71, pp. 347 - 352

[Non-patent Reference 6] Fox RI, "Sjogren's Syndrome: Evolving therapies", Expert Opinion in Investigative Drugs, 2003, 12, pp. 247 - 254

[Non-patent Reference 7] Johnson JT et al., "Oral Pilocarpine for Post-Irradiation Xerostomia in Patients with Head and Neck Cancer", The New England Journal of Medicine, 1993, 329, pp. 390 - 395

[Non-patent Reference 8] Fox RI et al., "Update in Sjogren Syndrome", Current Opinion in Rheumatology, 2000, 12, pp. 391 - 398

[Non-patent Reference 9] Atkinson JC et al., "Salivary Enhancement: Current Status and Future Therapies", Journal of Dental Education, 2001, 65, 10, pp. 1096 - 1101

[Non-patent Reference 10] Eneroth CM et al., "Effect of Fractionated Radiotherapy on Salivary Gland Function", Cancer, 1972, 30, pp. 1147 - 1153

[Non-patent Reference 11] Sheppard JD, "Guidelines for the Treatment of Chronic Dry Eye Disease", Managed Care, 2003, 12, 12 Supplement, pp. 20 - 25

[Non-patent Reference 12] Fox RI et al., "SHORT ANALYTICAL REVIEW, Use of Muscarinic Agonists in the Treatment of Sjogren's Syndrome", Clinical Immunology, 2001, 101, 3, 249 - 263

[Non-patent Reference 13] Zimmerman RP et al., "Concomitant Pilocarpine During Head and Neck Irradiation is Associated with Decreased Posttreatment Xerostomia", International Journal of Radiation Oncology Biology Physics, 1997, 37, 3, pp. 571 - 575

[Non-patent Reference 14] Coppes RP et al., "Muscarinic Receptor Stimulation Increases Tolerance of Rat Salivary Gland Function to Radiation Damage", International Journal of Radiation Biology, 1997, 72, 5, pp. 615 - 625
[Non-patent Reference 15] Kikuchi K el al., "Effect of Sialagogues on the Synthesis of Polyamines and DNA in Muraine Parotid Gland", Biochemical and Biophysical Research Communications, 1987, 144, 3, pp. 1161 - 1166

[Disclosure of the Invention]

[Problems that the Invention is to Solve]

Accordingly, an object of the invention is to provide a pharmaceutical composition for the treatment of tear and salivary fluid drying, which minimizes sweating and the like undesirable actions, and another object of the invention is to provide a pharmaceutical composition for selective tear and salivary fluid secretion acceleration, which minimizes sweating and the like undesirable actions and accelerates secretion of tear and salivary fluid.

[Means for Solving the Problems]

The present inventors have conducted extensive studies on the secretion action of tear and salivary fluid and growth action of lacrimal gland and salivary gland, related to muscarinic receptor agonists, and further on the avoidance of their undesirable actions which occur simultaneously with desirable actions.
When subcutaneous bolus administration was carried out on pilocarpine and cevimeline which are effective in improving dry mouth, the inventors have found that a higher dose than that for accelerating saliva secretion is necessary for the purpose of attaining tear fluid secretion acceleration, and that strong sweating action cannot be avoided by this dose.
Accordingly, sustained release of pilocarpine and cevimeline was used to avoid their sweating action, but similar to the case of subcutaneous bolus administration, it was not able to sufficiently reduce their sweating action but only finding secretion of salivary fluid.
When these findings and the aforementioned conventional techniques are taken into consideration, it seemed that in the case of accelerating both tear and salivary fluid secretion, a far larger one time dose than that for the acceleration of the salivary fluid secretion alone is required, and also, when taken into consideration that sweating action by sweat gland stimulation can not be avoided at this dose and the avoidance of sweating action by sustained release of pilocarpine and cevimeline does not sufficiently exert the effect, it was considered that solution of the problem of the invention by muscarinic receptor agonist is considerably difficult.
Under such a situation, when subcutaneous bolus administration of the compound A was carried out using mice and its actions were further examined, it was revealed that it accelerates secretion of tear and salivary fluid without accompanying sweating action, overturning the expectation as before. In addition, when the compound A was continuously administered to mice, it was found further to our surprise that this accelerates further secretion of not only salivary fluid but also tear fluid while sufficiently keeping sweating action low, and also accelerates glandular cell growth of salivary gland and lacrimal gland, thus resulting in the accomplishment of the invention.
That is, the invention relates to:

1. a pharmaceutical composition for treatment of tear and salivary fluid drying, which comprises (-)-(S)-2,8-dimethyl-3-methylene-1-oxa-8-azaspiro[4.5]decane or a pharmaceutically acceptable salt thereof as the active ingredient,
2. the pharmaceutical composition described in the aforementioned 1, wherein the active ingredient is L-tartarate monohydrate of the compound described in the aforementioned 1,
3. the pharmaceutical composition described in the aforementioned 1, which has a selective tear and salivary fluid secretion acceleration action,
4. the pharmaceutical composition described in the aforementioned 1 or 3, which has a glandular cell growth action,
5. the pharmaceutical composition described in the aforementioned 1 or 3, which has a form of sustained release preparations,
6. the pharmaceutical composition described in the aforementioned 5, which comprises the compound described in the aforementioned 1, or a pharmaceutically acceptable salt thereof, and a sustained release pharmaceutical carrier, and
7. the pharmaceutical composition described in the aforementioned 2, whose applicable disease is rheumatism, autoimmune diseases, medical diseases, atrophy of salivary gland and lacrimal gland due to aging, allergic keratitis and conjunctivitis, viral diseases, salivary gland and lacrimal gland disorders due to radiation irradiation, aging, psychological fatigue and dryness caused by a side effect at the time of drug administration.

The invention also relates to the use of the compound A described in the aforementioned 1 as a selective tear and salivary fluid secretion accelerator or a pharmaceutically acceptable salt thereof, wherein the compound A described in the aforementioned 1, or a pharmaceutically acceptable salt thereof, is L-tartarate monohydrate of the compound A, and to the use of the compound A described in the aforementioned 1 as a selective tear and salivary fluid secretion accelerator or a pharmaceutically acceptable salt thereof, which is administered in the form of sustained release preparations.

Also, the invention relates to the use of the compound A described in the aforementioned 1 or a pharmaceutically acceptable salt thereof for producing a selective tear and salivary fluid secretion accelerator, to the use of the compound A described in the aforementioned 1 or a pharmaceutically acceptable salt thereof for producing a selective tear and salivary fluid secretion accelerator, wherein the compound A described in the aforementioned 1, or a pharmaceutically acceptable salt thereof, is L-tartarate monohydrate of the compound A, to the use of the compound A described in the aforementioned 1 or a pharmaceutically acceptable salt thereof for producing a selective tear and salivary fluid secretion accelerator, wherein the adaptive diseases are rheumatism, autoimmune diseases and salivary gland and lacrimal gland disorders due to radiation irradiation, and further to the use of the compound A described in the aforementioned 1 or a pharmaceutically acceptable salt thereof for producing a selective tear and salivary fluid secretion accelerator which is administered as sustained release preparations.

The invention further relates to a method for treating a disease which requires acceleration of tear and salivary fluid secretion, that comprises administering an effective amount of the compound A described in the aforementioned 1 or a pharmaceutically acceptable salt thereof to a patient of dry eye and dry mouth, to the aforementioned therapeutic method, wherein the aforementioned patient of dry eye and dry mouth has rheumatism, an autoimmune disease or a salivary gland and lacrimal gland disorder due to radiation irradiation, to the aforementioned therapeutic method, wherein the compound A described in the aforementioned 1, or a pharmaceutically acceptable salt thereof, is L-tartarate monohydrate of the compound A, and further to the aforementioned therapeutic method which comprises administering as sustained release preparations.

The invention still further relates to a pharmaceutical composition for tear and salivary fluid secretion acceleration use, wherein the embodiment of sustained release preparations are prepared in such a manner that effective concentration of the compound A in plasma does not exceed about 2760 ng/ml, and the effective concentration of the compound A in plasma is maintained at from about 87 ng/ml to about 2760 ng/ml for at least 4 hours or more among the period of time until next administration, and to a pharmaceutical composition for tear and salivary fluid secretion acceleration use, wherein the ratio of the maximum concentration of the compound A in plasma after administration of the compound A ($C_{max}$) to the concentration of the compound A in plasma just before the next administration of said sustained release preparations ($C_{min}$), ($C_{max/cmin}$ ratio), shows about 91 or less.

The compound A according to the invention is a muscarinic receptor agonist which is an optically active substance (S isomer) having asymmetric carbon at the 2-position. The compound A of the invention forms an acid addition salt. Such salt is a pharmaceutically acceptable salt, and its preferred examples include acid addition salts with hydrochloric acid, hydrobromic acid, hydroiodic acid, sulfuric acid, nitric acid, phosphoric acid and the like inorganic acids and with formic acid, acetic acid, propionic acid, oxalic acid, malonic acid, succinic acid, fumaric acid, maleic acid, lactic acid, malic acid, tartaric acid, citric acid, methanesulfonic acid, ethanesulfonic acid, aspartic acid, glutamic acid and the like organic acids. In addition, the invention also includes various hydrates, solvates and polymorphic substances of the acid addition salts of the compound A.

The "tear and salivary fluid drying" according to the invention means mainly rheumatic and autoimmune diseases such as rheumatoid arthritis, Sjogren syndrome and systemic lupus erythematosus, medical diseases such as diabetes mellitus, hepatic cirrhosis and kidney failure, atrophy of salivary gland and lacrimal gland due to aging, allergic keratitis and conjunctivitis, viral diseases such as AIDS, salivary gland and lacrimal gland disorders due to radiation irradiation in cancer therapy, aging, psychological fatigue and the like, and also, dryness caused by undesirable actions at the time of the administration of various drugs including anti-hypertensive drug, antidepressant, antispasmodic agent, diuretic, muscle relaxant, anti-psychotic drug, anorectic and antiparkinsonism drug. Generation of the "tear and salivary fluid drying" by these diseases is described in various references (already described [Non-patent Reference 1], [Non-patent Reference 6], [Non-patent Reference 7], [Non-patent Reference 11] and the like).

Alleviation and treatment of symptoms of said drying become possible by accelerating secretion of tear and salivary fluid. In addition, since growth of lacrimal gland and salivary gland cells can also be expected, effective alleviation and treatment of symptoms become possible for diseases including exocrine gland disorders as the cause, among the aforementioned diseases.

The "selective tear and salivary fluid secretion acceleration" according to the invention means that, in the secretion of tear fluid, salivary fluid and sweat, particularly the secretion of tear and salivary fluid is selectively accelerated without accompanying secretion of sweat, and regarding the glandular cell growth, for example, it means separation of lacrimal gland and salivary gland cell growth and the like main actions and sweat gland cell growth and the like undesirable actions. That is, this means that, among the secretion actions of sweat, tear fluid and salivary fluid, expression of sweating action is controlled to a low level, while secretion of tear and salivary fluid is accelerated. For example, in the case of general pharmaceutical preparations showing general drug dissolution, this means that the dose or administration rate showing the sweating action is separated preferably by a factor of approximately from 2 to 3 times or more from the dose or administration rate which accelerates secretion of tear and salivary fluid, and in the case of sustained release pharmaceutical preparations, separated by a factor of preferably from 4 to 8 times or more.

In this connection, secretion of tear and salivary fluid is recognized as the effect when the invention is used, but its use for the purpose of tear fluid secretion alone or salivary fluid secretion alone can also be considered from the therapeutic point of view, so that the invention includes its use for the purpose of tear fluid secretion alone or salivary fluid secretion alone from the therapeutic point of view.

The "glandular cell growth" according to the invention indicates a phenomenon which is essential for maintaining glandular tissue and means glandular cell division for supplementing turnover of glandular cells or cell death induced by a certain morbid cause. This does not always indicate increase of the number of cells of the exocrine gland tissue. In addition, the glandular cell growth acceleration means maintenance or regeneration of exocrine gland as a result of accelerating glandular cell division by various stimulations, which results in the improvement and acceleration of external secretion function. Such a function is directly verified morphologically and biochemically, such as biopsy and pathologic inspection of exocrine gland or exocrine gland scintigraphy. It is possible also to indirectly verify based on external secretion ability.

The "possessed of glandular cell growth" means that it shows glandular cell growth at a dose or administration rate which does not shows sweating action, and preferably, this means the "glandular cell growth" when the indexes in the measuring systems of sweating action and ornithine decarboxylase activity of glandular tissue, shown in Examples, are used.

The invention is realized by administering the compound A or a medically acceptable salt thereof to a patient showing a symptom of dry mouth and eye. In addition, it is possible to obtain further useful effect by gradually administering it or gradually releasing it in the living body.

The "sustained release" according to the invention means slow administration of the compound A or its gradual release from a pharmaceutical preparation.

The "slowly administer" or "gradually release" means that a drug contained in a pharmaceutical preparation is administered or released, for example, during a period of from 2 hours to 24 hours, preferably from 3 hours to 24 hours, more preferably from 5 to 24 hours. Illustratively, it means that a predetermined amount of the compound A is administered or released during a predetermined period of time.

The "sustained release pharmaceutical preparation form" according to the invention means that it is a pharmaceutical preparation having the aforementioned property of "sustained release", and its various illustrative embodiments are minutely exemplified in this specification.

Though it is possible to obtain tear and salivary fluid secretion acceleration effect without sweating acceleration action by general administration, in order to obtain effects of further sufficient increase of tear and salivary fluid secretion, and lacrimal gland and salivary gland cell growth, it is desirable to control drug release per unit hour of the compound A from a sustained release pharmaceutical preparation. Regarding the drug release rate, there is a possibility that it varies depending on the influences of specific difference, individual difference and the like various factors, but for example, the drug release rate can be roughly calculated by making use of the following human clinical test results which use compound A-containing standard oral capsules and animal test results at the time of bolus administration and continuous administration of compound A.

The dose of compound A by which the effect of glandular cell growth was observed at continuous administration (24 h/once) to mice is from 100 mg/kg to 400 mg/kg (2.5 mg/individual to 10 mg/individual, based on the assumption of 25 g mouse body weight/individual, calculated by the formulae of 2.5/0.025 and 10/0.025 based on the above relation), and the drug infusion (release) rate by which the effect was obtained in mouse is from 4.2 mg/kg/h to 16.7 mg/kg/h (calculated from 100/24 and 400/24) (I).

In addition, the drug infusion (release) rate by which the effect was not obtained in mouse is 2.1 mg/kg/h and 33.3 mg/kg/h (calculated from 1.25/0.025/24 and 20/0.025/24) (II). The drug release rate by which the effect of glandular cell growth could be expected by continuously administering the compound A to human was calculated by extrapolating the aforementioned values of drug release rate in mouse (I and II) to human.

Based on the results of human clinical test and at bolus administration to mice, the extrapolation values to human was applied flexibly, by defining the dose at which a side effect was found as 60 mg/individual in the case of human and 30 mg/kg in the case of mouse, the dose at which a side effect was not found as 40 mg/individual in the case of human and 10 mg/kg in the case of mouse, and the human body weight as 60 kg, and thereby using 60/60/30 = 1/30 and 40/60/10 = 1/15, respectively as the factors for extrapolating to human. When the value of I and 1/30 are used as the factors, from 8.3 mg/h/individual to 33.3 mg/h/individual is one of the values of administration rate by which the effect can be found, based on 4.17/30 * 60 and 16.67/30 * 60. Also, when the value of II and 1/30 are used as the factors in the same manner, it is from 4.2 mg/h/individual to 66.7 mg/h/individual, based on 2.08/30 * 60 and 33.33/30 * 60. Further, when calculated from I using 1/15 as the factor in the same manner, it is from 16.7 mg/h/individual to 66.7 mg/h/individual, based on 4.17/15 * 60 and 16.67/15 * 60. In addition, from II and a factor 1/15, it is from 8.3 mg/h/individual to 133.3 mg/h/individual, calculated from 2.08/15 * 60 and 33.33/15 * 60.

Based on the above values, to define as from 4.2 mg/h/individual to 133.3 mg/h/individual is one of the desirable embodiments of the sustained release pharmaceutical preparations according to the invention. Also, to define as from 8.3 mg/h/individual to 133.3 mg/h/individual is a further desirable embodiment. When these doses are converted into daily dose, it becomes from 100 to 3200 mg. From 200 to 3200 mg is a further desirable daily dose.

Based on the phase I study (oral administration) of the compound A shown in the following, the minimum dose by which increase of the salivary fluid secretion was observed is 10 mg (Table 7). The value of Cmax in that case was $35.3 \pm 10.5$ (ng/ml) (Table 8). Accordingly, it can be considered that the tear fluid and saliva secretion action will be observed when its plasma concentration is 50 ng/ml. When it is assumed that the 50 ng/ml is maintained for 24 h, the AUC becomes 50 ng/ml * 24 h = 1200 ng·h/ml, which approximates the 1010 ng·h/ml at the time of the 40 mg oral administration when compared with the value of AUC of Table 8. Accordingly, the dose for maintaining the aforementioned 1200 ng·h/ml is 400 mg.

On the other hand, regarding the maximum dose (single administration) by which sweating was not observed but the beneficial effect was observed, this was at the time of 40 mg administration, and since the Cmax in that case was $151.0 \pm 24.1$ (as 150 ng/ml) which was 3 times of the aforementioned 50 ng/ml, this can be calculated as 4- mg * 3 = 120 mg (Tables 7 and 8). The dose as a pharmaceutical preparation is optionally decided in response to individual case by taking symptoms and age, sex and the like of each subject to be administered into consideration, but when deduced from the aforementioned dose calculated from the drug release rate, this is 10 mg at the lowest (phase I study result, Table 7), preferably 20 mg, more preferably 40 mg, per day per adult in the case of standard administration. The maximum dose can also be selected in the same manner in response to individual case, but is 3200 mg, preferably 1500 mg, more preferably 500 mg, further preferably 250 mg, and most preferably 120 mg. In this connection, these description on these doses and the following description on the doses are examples calculated based on its tartaric acid salt, and these doses can be optionally converted also on its free form and other salts.

Also, the drug release rate, when 24 h release from the lower limit value 40 mg of the aforementioned most preferred dose is taken into consideration, can be calculated as 40 mg/24 h = 1.7 mg. The slow drug release rate calculated in the above was 4.2 mg/h/individual, and by comparing with this value, the available most slow drug release rate can be set to 1.7 mg/h/individual.

In addition, it is possible also to control drug release ratio per 1 hour of the compound A. For example, the release rate when the drug is released 100% in 24 h (one administration per day) can be set to about 4%/h. On the other hand, since Tmax was about 2 h in the human test result which used general tablets containing the compound A, the drug release rate can be set to about 50%/h (100% release in 2 h). Since there is a possibility that it varies depending on the influences of individual difference and the like various factors, various release rate can be set, but for example, it is desirable that release rate of the compound A is from about 4%/h to about 50%/h.

As described in the above, in order to increase secretion of salivary and tear fluid and to obtain effect of salivary gland and lacrimal gland cell growth, more sufficiently than usual, it is desirable to control drug release per unit hour of the compound A from a sustained release pharmaceutical preparation, and essentially, it is ideal to keep its effective concentration in human plasma for a predetermined period of time. The effective concentration of compound A in plasma under stationary state can be calculated using the AUC and disappearing half life obtained in the human clinical test, the aforementioned drug release per unit hour, distribution volume, disappearing rate constant and the like pharmacokinetic parameters.

An example of the method for calculating its concentration in plasma under stationary state (Css) is shown in the following.

$$\text{Calculation formula: } Css = R/(Vd * Kel)$$

$$Kel = 0.693/T1/2$$

$$Vd = dose/AUC/Kel$$

R (mg/h): drug release per unit hour
Vd (ml): distribution volume
Kel (1/h): disappearing rate constant
T1/2: disappearing half life

From the results of P-I (Table 7), among the doses of 10, 20 and 40 mg by which sweating was not found but salivary fluid secretion was observed, data on AUC (410 ng * h/ml) and T1/2 (3.38 h) at the time of the central value 20 mg administration were used, and Css was calculated based on the aforementioned formula by setting desirable release rate (R) to R = 4.2, 8.3, 66.7 or 133.3 mg/h. They were 86.9, 172, 1380 and 2758 ng/ml. Though there is a possibility that it varies depending on the influences of specific difference, individual difference and the like various factors, when an example is cited, effective concentration of the compound A in plasma is 35 ng/ml in a lower case, based on the aforementioned calculation result and from the aforementioned valued calculated from Tables 7 and 8. This is preferably 50 ng/ml, more preferably 87 ng/ml, and further more preferably 170 ng/ml. On the other hand, this is 2760 ng/ml and preferably 1380 ng/ml, in a higher case.

The period of time for keeping the aforementioned effective concentration in plasma can be calculated based on the results of human clinical test, by simulating PK profile and PK parameters at the time of the continuous administration of standard tablet containing the compound A. For example, it is possible to use WinNonlin (Ver 2.1, Pharsight, USA) or the like calculation software for the calculation of various parameters. Though there is a possibility that it varies depending on the influences of individual difference and the like various factors, for example, it is desirable to keep the effective concentration in plasma for 4 hours or more, preferably 6 hours or more.

In addition, it is desirable to control a PT ratio (Cmax/Cmin) which is the ratio of maximum concentration of the compound A in plasma (Cmax) to its minimum concentration in plasma (Cmin). It is possible to calculate the PT ratio from Tmax and T1/2, and the calculation is carried out in the following manner.

From the results of P-I, 20 mg administration, (24 - 2)/4 = 5.5(II - I/III) is calculated using a period of time (II) from in and after Tmax (I) to the next administration (supposing once a day administration) and a value of T1/2 (III, round off the average value of those which were effective). This value means that the hour of T1/2 passed 5.5 times after Tmax, and $(1/2)^{5.5} = 1/45$. Thus, the PT ratio becomes 45.

Also, when supposed as twice a day administration, (12 - 2)/4 = 2.5. In the same manner as in the above, $(1/2)^{2.5} = 1/5.7$. Thus, the PT ratio is 5.7. In addition, when the same calculation is carried out based on the minimum value 3.4 h of T1/2 (showing the effect without perspiration),

$$(24 - 2)/3.4 = 6.5, \quad (1/2)^{6.5} = 1/90.5,$$

and

$$(12 - 2)/3.4 = 2.9, \quad (1/2)^{2.9} = 1/7.5.$$

Based on the above, an example of the PT ratio is about 91 or less, preferably about 45 or less, and more preferably about 7.5 or less.

The lacrimal gland and salivary gland cell growth can be also associated with the tear and salivary fluid secretion according to the invention. Though its mechanism is not clear yet, as shown in Examples, not only the tear and salivary fluid secretion but also the lacrimal and salivary gland cell growth is accelerated by the pharmaceutical composition having a sustained release pharmaceutical preparation form which is indicated in the present invention, so that it becomes possible to induce regeneration and repair of the damaged glandular tissues.

Since the compound A is a muscarinic receptor agonist and because of the various pharmacological actions well known by this drug, there was a worry about the narrowness between its blood concentration for accelerating tear and salivary fluid secretion and its blood concentration for expressing undesirable pharmacological actions in other tissues. However, it became possible by the invention to provide a therapeutic method which is far better than the conventional techniques for alleviating dry mouth and dry eye, by attaining acceleration of tear and salivary fluid secretion without accompanying sweating action, and also by stimulating the salivary gland and lacrimal gland selectively.

In general, an oral administration preparation having general dissolution property shows a high blood concentration at the initial stage of its administration, and the blood concentration of the drug is gradually reduced thereafter to its effective blood concentration or less, and its therapeutic effect disappears. One of the further important points of the invention is that, when a sustained release pharmaceutical preparation form is selected, high blood concentration at the initial stage of its administration can be avoided because of the controlled drug release rate, thus it becomes possible to provide much better treatment of dry mouth and dry eye than usual.

The route of administration for attaining the effect of the invention is not especially limited if it shows the effect of the invention. Its examples include oral preparations, injections, drip infusions, transdermal preparations, ointments and the like external preparations, rectal suppositories, vaginal suppositories and the like suppositories, and pellets and the like parenteral preparations. These can be easily prepared by generally known preparation methods, and it is possible to select recipes suited for various administration methods.

In addition, in the case of employing a sustained release pharmaceutical preparation form to obtain further beneficial effect, there is no especial limitation if it has a sustained release property. Preferably, an oral sustained release pharmaceutical composition (oral sustained release pharmaceutical preparation) or a sustained release pharmaceutical preparation for injection (e.g., subcutaneous, intramuscular, intraperitoneal or the like) is employed, but other delivery systems can also be usable. For example, they are drip infusions, transdermal preparations, ointments and the like external preparations, rectal suppositories, vaginal suppositories and the like suppositories, and pellets and the like parenteral preparations.

When a pharmaceutical composition of oral sustained release pharmaceutical preparation form is employed in the invention, it is possible to employ various embodiments for the purpose of attaining a planned fixed drug release per unit hour showing the sustained release property of the invention, and for example, the embodiments shown in the following can be exemplified.

(A) Sustained release hydrogel-forming pharmaceutical preparation

The carrier to be used for sustained release pharmaceutical preparations comprises an additive agent for effecting permeation of water into the inner part of the pharmaceutical preparation (also called a gelling agent, gelling accelerator or hydrophilic base, but this is to be referred to as "hydrophilic base" hereinafter) and a high molecular substance for hydrogel formation (hydrogel-forming high molecular substance). As the sustained release hydrogel-forming pharmaceutical preparation, for example, those which are described in an international pamphlet WO 9406414, an international pamphlet WO 0110466, an international pamphlet WO 0178686, an international pamphlet WO 2003041656, an US pamphlet US 6436441, an US pamphlet US 6562375 an US pamphlet US 20030203024, an US pamphlet US 20040091528 and the like can be cited, and these are included in the contents of the invention.

As the method for producing such a sustained release pharmaceutical composition preparation, there is no particular limitation with the proviso that it is a method which can produce general hydrogel-forming pharmaceutical preparations. As an example thereof, for example, a tabletting method in which a drug, a hydrophilic base (e.g., polyethylene glycol (trade name PEG 6000 (mfd. by NIPPON OIL & FATS)), polyvinyl pyrrolidone (trade name PVPK 30, mfd. by BASF, or the like), D-sorbitol, xylitol and the like sugar alcohols, and a hydrogel-forming high molecular substance (e.g., polyethylene oxide (PEO) (trade name Polyox WSR-303 (average molecular weight: 7,000,000, viscosity: 7500 - 10000 cps (1% aqueous solution 25°C) or the like)) or the like), and further yellow ferric oxide and/or red ferric oxide and the like additive agents as occasion demands, are added and mixed and subjected to compression molding, a capsule compression filling method thereof, or extrusion molding or injection molding method in which the mixture is melted and then formed by solidifying it, and the like can be cited. In addition, a general sugar coating, film coating or the like coating treatment can also be applied after the molding. Alternatively, the material after the molding may be filled in capsules.

Their blending amounts are fully disclosed in the aforementioned references, but in exemplifying them, the hydrophilic base is approximately from 5 to 80 W/W %, preferably from 5 to 60 W/W %, the hydrogel-forming high molecular substance is from 10 to 95 W/W %, preferably from 15 to 90 W/W %, and yellow ferric oxide and/or red ferric oxide is from 1 to 20 W/W %, preferably from 3 to 15 W/W %, based on the whole pharmaceutical preparation.

Regarding this pharmaceutical preparation, it is possible to freely adjust its sustained release period of time, drug release rate and the like by changing the aforementioned composition, for example, by changing blending ratio of the hydrogel-forming high molecular substance and hydrophilic base or their blending amounts.

(B) Osmotic pump type pharmaceutical preparation:

The osmotic pressure pump type pharmaceutical preparation is a pharmaceutical preparation in which a semi-permeable membrane through which water and external liquid are permeable, but a drug, a osmotic pressure agent, an osmopolymer and the like are not permeable, is coated on a double layer tablet type compressed core consisting of a drug layer containing a drug or a pharmaceutically acceptable salt thereof (preferably hydrochloride) and a push layer. At least one drug delivery orifice is arranged on the semi-permeable membrane in order to connect internal and external environments of the pharmaceutical preparation. Accordingly, the osmotic pressure pump type pharmaceutical preparation has a mechanism in which, after this is orally ingested, water and the like liquids permeate through the semi-permeable membrane and infiltrate into inner part of the pharmaceutical preparation, and the drug is continuously released through the drug delivery orifice by the thus generated osmotic pressure action, at a constant rate for a prolonged period of time even in an environment having different pH value.

This pharmaceutical preparation is reported in "Osmotic drug delivery: a review of the patent literature", edited by Santus and Baker, and Journal of Controlled Release, 35, pp. 1 - 21 (1995). Also, this pharmaceutical preparation is described in US Patent 3,845,770 specification, US Patent 3,916,899 specification, US Patent 3,995,631 specification, US Patent 4,008,719 specification, US Patent 4,111,202 specification, US Patent 4,160,020 specification, US Patent 4,327,725 specification, US Patent 4,519,801 specification, US Patent 4,578,075 specification, US Patent 4,681,583 specification, US Patent 5,019,397 specification and US Patent 5,156,850 specification, and all of the contents described in said specifications are incorporated into this specification.

The drug layer is constructed from a pharmaceutical composition comprising a carrier for sustained release pharmaceutical composition use which comprises a pharmacologically effective amount of a drug for treatment or prevention or a pharmaceutically acceptable salt thereof and a hydrophilic polymer, such as poly(ethylene oxide)as a poly(alkylene oxide) having a number average molecular weight of from 100,000 to 750,000 or the like, which releases the drug at a constant releasing rate. In addition, this can contain a hydroxypropyl alkyl cellulose having a number average molecular weight of from 9,200 to 125,000, typically hydroxypropylethylcellulose or the like, for the purpose of improving delivery characteristics of the pharmaceutical preparation, and poly(vinyl pyrrolidone) having a number average molecular weight of from 7,000 to 75,000 for the purpose of improving fluidity of the pharmaceutical preparation. Blending ratio of the hydrophilic polymer to be used is influenced by factors such as the physicochemical characteristics, the content and the like of the drug to be contained, but is from 40 to 90 W/W % to the weight of the drug layer.

In order to extrude a drug or a pharmaceutically acceptable salt thereof through the outlet of the pharmaceutical preparation, it is possible to contain a component selected from an osmopolymer which swells by absorbing an aqueous liquid or a body fluid (a polymer having the action to highly swell or expand by interacting with water or a biological liquid), such as a poly(alkylene oxide) having a number average molecular weight of from 1,000,000 to 15,000,000 typified by polyethylene oxide and the like, in the push layer. Blending amount of the "osmopolymer" is influenced by the characteristics, content and the like factors of the drug in the drug layer, but is for example 30 mg or more, preferably 50 mg or more. The blending ratio is from 40 to 80 W/W % to the weight of the push layer.

Regarding the osmotic pressure agent to be used, this may be contained in both layers of the drug layer containing a drug or a pharmaceutically acceptable salt thereof and the push layer, and there is no particular limitation with the proviso that it shows an osmotic pressure gradient via the semi-permeable membrane. As such an osmotic pressure agent, one or two or more of inorganic salts or organic salts selected from sodium chloride and the like can be exemplified. Blending ratio of the osmotic pressure agent is from 15 to 40 W/W % to the weight of the push layer.

The semi-permeable polymer is described in US Patent No. 4,077,407, and said polymer can be obtained by synthesizing it by the method described in Encyclopedia of Polymer Science and Technology, vol. 3, pp. 325 - 354 (1964), Interscience Publishers, Inc., New York, NY. Blending ratio of the polymer to be used is not particularly limited, with the proviso that it is such an amount that permeability of water, living body fluid and the like external liquids is high, but permeability of the drug, osmotic pressure agent, osmopolymer and the like can be regarded as substantially unpermeable, but is preferably from 6 to 20 W/W %, more preferably from 8 to 18 W/W %, to the weight of the double layer compressed core consisting of the drug layer and push layer.

Said sustained release pharmaceutical composition is prepared by a conventionally known method and can be prepared with reference to the aforementioned various US patents, and US Patent No. 3,916,899 specification, US Patent No. 4,088,864 specification or the like on the device for forming the outlet.

In this pharmaceutical preparation, it is possible to provide a desired releasing rate by the coating amount of the semi-permeable membrane, blending amount of osmopolymer in the push layer and molecular weight (viscosity) of hydrophilic polymer in the drug layer. Regarding the blending amounts, various polymers, fillers and the like, they are described in detail in the aforementioned references and the like and can be easily prepared thereby.

(C) Gel pharmaceutical preparation in which two or more gums are combined

The carrier to be used for sustained release pharmaceutical composition use comprising a sustained release filler consisting of a hetero polysaccharide gum and a homo polysaccharide which can cross-link said hetero polysaccharide gum when applied to an environmental fluid, an inert diluent selected for example from a monosaccharide, a disaccharide and a polyhydric alcohol, or a mixture thereof, and a pharmaceutically acceptable water-soluble cationic crosslinking agent for providing a sustained drug release property for at least about 24 hours when the drug dose is applied to an environmental fluid. In addition to blood, gastrointestinal fluid and the like body fluids, for example, aqueous solutions which are used for *in vitro* dissolution tests are also included in the "environmental fluid".

As described in US Patent No. 4,994,276 specification, US Patent No. 5,128,143 specification and US Patent No. 5,135,757 specification, it is known that a hetero-dispersing filler comprising a synergism-showing combination of hetero polysaccharides and homo polysaccharides, such as a combination of two or more of polysaccharide gums, has a viscosity higher than that of either gum alone, forms quick hydration, and the thus formed gel is further quickly formed and becomes further hard.

As the aforementioned sustained release pharmaceutical composition, this is produced, for example, as a pharmaceutically acceptable oral solid drug dose form such as a tablet. In citing an example, (1) a hetero polysaccharide gum and a homo polysaccharide which can cross-link said hetero polysaccharide gum when applied to an environmental fluid are dry-mixed together with a pharmaceutically acceptable inert diluent at a desired ratio, (2) the mixture of these is subjected to wet granulation, (3) the granulated granules are dried, and (4) the dried granules are pulverized to obtain a sustained release filler having a desired particle diameter, and then this sustained release filler is (5) subjected to granulation together with a drug or a pharmaceutically acceptable salt thereof, (6) the thus formed granules are dried, and subsequently, (7) an inert filler (e.g., a lubricant) is added thereto, and said mixture is then, for example, (8) compression-molded into tablets.

In an example of the optimum combination of respective components, xanthan gum as the "hetero polysaccharide" and locust bean gum as the "homo polysaccharide" are blended at a ratio of about 1:1 in an amount of from about 35 to about 50 W/W % of, to the total weight of the sustained release pharmaceutical composition, and about 10 W/W % or less of calcium sulfate as the "water-soluble cationic crosslinking agent", about 35 W/W % of dextrose as the "inert diluent" and from about 5 to about 10 W/W % of ethyl cellulose as the "hydrophobic substance" are further blended.

According to this pharmaceutical preparation, it becomes possible to provide a sustained release pharmaceutical preparation having a desired releasing rate, by adjusting blending amounts of the homo polysaccharides and hetero polysaccharides and their blending ratio.

(D) Multi-layered tablet pharmaceutical preparation comprising geometrically arranged drug nucleus and release controlling layer(s)

The carrier to be used for sustained release pharmaceutical composition comprises a layer which contains a drug and release controlling layer(s), and comprises the following construction:

a sustained release pharmaceutical composition comprising two or three layers (compression-molded product such as tablet), characterized in that it comprises

a) a first layer (layer 1) which contains a water-soluble polymer and has a property to swell when contacted with an environmental fluid,
b) a second layer (layer 2) which contains a drug or a pharmaceutically acceptable salt thereof (suitably hydrochloride) and is arranged such that it adjoins the first layer and releases a physiologically active substance within a period of time determined in advance, and
c) as occasion demands, a water-soluble polymer which generally gells and/or swells and then optionally disintegrates, and also a third layer (layer 3) attached to the layer 2. In addition to blood, gastrointestinal fluid and the like body fluids, for example, aqueous solutions which are used for dissolution tests are also included in the "environmental fluid".

As described in US Patent No. 4,839,177 specification and US Patent No. 5,422,123 specification, the aforementioned sustained release pharmaceutical composition is characterized in that releasing rate of a drug from the pharmaceutical preparation is controlled by interposing the layer 2 containing the drug between the layer 1 and layer 3 which do not contain or optionally contain the drug.

Also, as described in US Patent No. 5,780,057 specification and US Patent No. 6,149,940 specification, it is known that said sustained release pharmaceutical composition has a function as follows, by a method in which at least one of the layer 1 and layer 3 rapidly swells by contacting with a body fluid and then the layer 2 swells in the same manner, that is, the volume of said pharmaceutical composition considerably increases, so that the pharmaceutical composition remains in the stomach for a more longer period of time and the majority of the active substance contained therein is dissolved and absorbed in a controlled manner in the upper part of the digestive tract.

The water-soluble polymer to be used in the layer 1, layer 3 and layer 2 is not particularly limited with the proviso that it is pharmaceutically acceptable and has biological compatibility. As such a water-soluble polymer, for example, a water-soluble cellulose derivative such as hydroxypropylmethylcellulose or the like can be cited, and its molecular weight is preferably from 3,000 to 2,000,000. Blending amount of the water-soluble polymer in the layer 1 and layer 3 is generally from 5 to 90 W/W %, preferably from 10 to 85 W/W %, more preferably from 20 to 80 W/W %, to its weight. Blending amount of the water-soluble polymer in the layer 2 is generally from 5 to 90 W/W %, preferably from 10 to 85 W/W %, to its weight.

Tablets comprising said sustained release pharmaceutical composition are prepared by a method in which powders and/or granules are mixed using a conventionally known production technique and subjected to compression molding, and the like. The pharmaceutical composition comprising two or three layers (e.g., tablets) can be prepared by a conventionally known tableting method. The tablets of the invention can be prepared, for example, using a rotary press which can produce "multi-layered" tablets.

According to this pharmaceutical preparation, it becomes possible to provide a sustained release pharmaceutical preparation having a desired releasing rate, based on the molecular weight of the water-soluble polymer to be used in the release controlling layer, thickness of the release controlling layer, addition of a hydrophobic substance to the release controlling layer, the water-soluble polymer content in the drug-containing layer and molecular weight thereof, thickness and geometrical shape of the drug-containing layer, and diameter size of the multi-layered tablets.

(E) Gastroretentive doasage form using swelling polymer

The carrier to be used for sustained release pharmaceutical composition comprises a high molecular weight water-soluble polymer which swells at the time of water absorption. Such a polymer can be used individually or in combination.

Said carrier for sustained release pharmaceutical composition use is described for example in US Patent No. 6,340,475 specification, US Patent No. 5,972,389 specification, US Patent No. 5,582,837 specification and US Patent No. 5,007,790 specification, and all of the contents described in the aforementioned specifications are incorporated into this specification.

The sustained release pharmaceutical composition is prepared as a pharmaceutically acceptable oral solid drug dose forms such as tablets, granules, particles which can be included in tablets or capsules, and the like. Presently desirable dosage forms are, for example, those in which 2 or 3 drug-containing polymer particles (pellets) are included in No. 0 gelatin capsule.

A granular drug/polymer mixture or a polymer matrix impregnated with a drug can be prepared by conventionally known methods by employing various mixing, pulverizing and manufacturing techniques. For example, direct compression using appropriate die and punch and injection or compression molding can be cited. A lubricant may be added at the time of compression molding.

An example of the optimum combination of the aforementioned respective components is to blend from about 90 to about 97 W/W % of a polyethylene oxide having a weight average molecular weight of within the range of from about 2,000,000 to about 7,000,000, to the total weight of the sustained release pharmaceutical composition, as the "high molecular weight water-soluble polymer which swells at the time of water absorption", and less than about 2 W/W % of magnesium stearate to the total weight of the sustained release pharmaceutical composition, as the "lubricant". Also, an example of the combination in which two species, for example, of water-soluble polymers are blended is to blend about 48 W/W % for each of a polyethylene oxide having a weight average molecular weight of within the range of from about 900,000 to about 7,000,000 and a hydroxypropylmethyl cellulose having a viscosity of from about 3 to about 10,000 cps as its 2% aqueous solution at 20°C, at a blending ratio of about 1:1.

According to this pharmaceutical preparation, it becomes possible to provide a sustained release pharmaceutical preparation having a desired releasing rate, by the molecular weight and blending amount of the water-soluble polymer and by a combination of two or more water-soluble polymers.

(F) Matrix pharmaceutical preparation using a water-soluble polymer

The matrix tablet which uses a water-soluble polymer is a sustained release pharmaceutical composition carrier in which a drug is uniformly dispersed in a water-soluble polymer base such as hydroxypropylmethylcellulose. Amount of the water-soluble polymer is from 5 to 95 W/W %, preferably from 10 to 90 W/W %, more preferably from 30 to 85 W/W %, per unit pharmaceutical preparation.

This matrix pharmaceutical preparation is described, for example, in International Publication No. 93/16686 pamphlet, and all of the contents described in the aforementioned specification are incorporated into this specification.

Hydroxypropylmethylcellulose as the water-soluble polymer undergoes hydration when contacted with water and forms a hydrogel layer on the tablet surface. A drug is released by the gradual dissolution and erosion of the drug-containing gel layer formed on the tablet surface. The tablets have a characteristic in that sustained release of a drug is achieved by repeating these contact with water, formation of gel layer containing a drug and dissolution and erosion of the gel layer.

The tablets comprising said pharmaceutical composition can be prepared by a conventionally known method. Such tablets can be prepared by a tabletting method which are used very generally and conventionally known by those skilled in the art.

According to this pharmaceutical preparation, it becomes possible to provide a sustained release pharmaceutical preparation having a desired releasing rate, by the molecular weight and blending amount of the water-soluble polymer.

(G) Drug diffusion-controlled type matrix pharmaceutical preparation

Said pharmaceutical preparation is a sustained release pharmaceutical composition carrier in which diffusion of a compound or drug having high solubility in water from the matrix is controlled. Said matrix pharmaceutical preparation is described in International Publication No. 2003/041656 pamphlet, and all of the contents described in the aforementioned specification are incorporated into this specification.

Said composition comprises a physiologically active substance (drug) having an electric charge and at least one polymer filler or polymer (counter polymer) having opposite charges to the physiologically active substance. When the physiologically active substance has positive charge, said composition can comprise a negatively charged polymer with carboxyl group, sulfate group or the like, and though not particularly limited, polymers comprising polyacrylic acid and sulfuric acid system are included in the particularly desirable polymers having negative charges. Carrageenan and dextran sulfate are included in the sulfuric acid system polymer. More preferably, when polyacrylic acid is selected as one polymer, a sulfuric acid system polymer can be selected as the other polymer.

Preferably, a hydrogel-forming polymer having such a physical characteristic that it shows high viscosity when gells can also be contained in the composition, and by this, the pharmaceutical preparation of the invention can withstand contraction movement of the digestive tract accompanied by the digestion of food and can maintain its shape more or less until it reaches the lower part of the digestive tract, namely the colon. For example, a polymer having a viscosity of 1000 cps or more as its 1% aqueous solution (at 25°C) is particularly desirable. Since characteristics of a polymer depend on its molecular weight, a substance of a relatively large molecular weight, namely an average molecular weight of 2,000,000, more preferably 4,000,000 or more, is desirable as said hydrogel-forming polymer.

In order to attain sustained release of a drug from the pharmaceutical preparation even at the lower part of human digestive tract similar to the case of the upper part of the digestive tract, a hydrophilic base may be further added to said composition. As said hydrophilic base, there is no particular limitation with the proviso that the hydrogel-forming high polymer to be used in said pharmaceutical composition can be dissolved before its gelation.

The drug release rate from said pharmaceutical composition can be controlled by the blending amount of the counter polymer and blending amount of the hydrogel-forming high polymer, and also by the combination of two or more counter polymers.

[Advantage of the Invention]

According to the method of the invention for treating dry mouth and dry eye, the orally or parenterally administered pharmaceutical composition consisting of the compound A accelerates secretion of tear and salivary fluid without accompanying side effects, by acting upon the muscarinic receptors of salivary gland and lacrimal gland. In addition, when a sustained release pharmaceutical preparation form is selected, it further accelerates secretion of tear and salivary fluid and also induces regeneration and repair of tissues by stimulating the muscarinic receptors of salivary gland and lacrimal gland tissues damaged by various causes, and thereby accelerating growth of the cells. Accordingly, pain of patients can be alleviated by preventing or treating dry mouth and dry eye accompanied by various diseases or caused by the treatment of diseases and also the dryness caused by mental fatigue, disorder and the like.

[Brief Description of the Drawings]

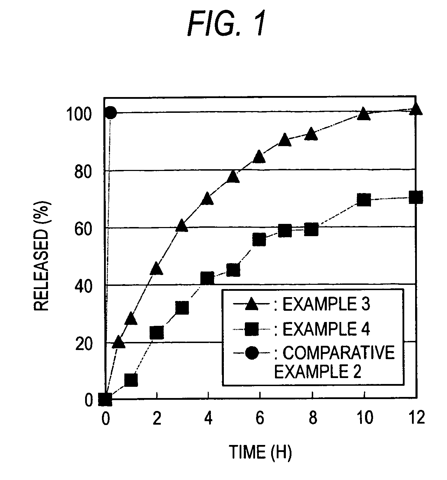

Fig. 1 is a graph showing a result of dissolution tests of Example 3, Example 4 and Comparative Example 2.

[Best Mode for Carrying Out the Invention]

The invention is described further in detail based on examples. The invention is not limited to these examples.

[Examples]

Test Example 1

I. Secretion of tear and salivary fluid and exocrine gland reaction by subcutaneous bolus administration of compound A and subcutaneous implantation of compound A-filled osmotic pump

Examination was carried out on the salivary and tear fluid secretion action, ornithine decarboxylase of salivary and lacrimal gland inducing action and sweating action, by subcutaneous bolus administration of compound A (Example 1) and continuous administration (Example 2) by subcutaneous implantation of a compound A-filled osmotic pump (Alzet mini-osmotic pump 2001 D, 8 μl/h, 1 day; DURECT Corporation).

Test Method

Male Balb/c mice were used in the test.

Example 1 Subcutaneous bolus administration

The compound A was dissolved in physiological saline to a concentration of 1, 3, 10, 30 or 100 mg/5 ml, and 5 ml/kg of the drug solution was administered under the dorsal skin. Secreted amounts of salivary and tear fluid were measured just before the administration of compound A and 10, 20, 30, 40 and 50 minutes after the administration. The sweating action was measured 10 minutes after the administration. Also, the growth acceleration action was measured 6 hours after the administration.

Example 2 Subcutaneous continuous administration

The compound A was dissolved in physiological saline to a concentration of 0.625, 1.25, 2.5, 5.0, 10 or 20 mg/ 200 μl, and filled each pump (Alzet mini-osmotic pump 2001D, 8 μl/h, 1 day; DURECT Corporation) with about 200 μl portions . It means, that is, each pump releases at a rate of 0.025, 0.05, 0.1, 0.2, 0.4 or 0.8 mg compound A/h, respectively. About 5 mm of dorsal epidermis of a mouse anesthetized with diethyl ether was incised, the osmotic pump already filled with the compound A was subcutaneously implanted, and then the skin was stitched. Secreted amounts of salivary and tear fluid and sweating were periodically measured from just before the implantation of pump until 32 hours after the administration. The ornithine decarboxylase activity was measured 10 hours after the implantation.

Comparative Example 1 Subcutaneous bolus administration of pilocarpine and cevimeline

Pilocarpine was dissolved in physiological saline to a concentration of 0.03, 0.1, 0.3, 1, 3 or 10 mg/5 ml, or cevimeline to a concentration of 0.3, 1, 3, 10, 30 or 100 mg/5 ml, and 5 ml/kg of the drug solution was administered under the dorsal skin. Secreted amounts of salivary and tear fluid were measured just before the administration of pilocarpine or cevimeline and 10, 20, 30, 40 and 50 minutes after the administration. The sweating action was measured 10 minutes after the administration. Also, the growth acceleration action was measured 6 hours after the administration.

Comparative Example 1 Subcutaneous continuous administration of pilocarpine and cevimeline

Pilocarpine was dissolved in physiological saline to a concentration of 0.039, 0.078, 0.16 or 0.31 mg/200 μl, or cevimeline to a concentration of 0.16, 0.31, 0.63 or 1.3 mg/200 μl, and filled the osmotic pump (Alzet mini-osmotic pump 2001 D, 8 μl/h, 1 day; DURECT Corporation) with about 200 μl portions. It means, that is, each pump releases at a rate of 0.00156, 0.00312, 0.0064 or 0.0124 mg/h (pilocarpine) or 0.0064, 0.0124, 0.0252 or 0.05 mg/h (cevimeline). About 5 mm of dorsal epidermis of a mouse anesthetized with diethyl ether was incised, the osmotic pump already filled with pilocarpine or cevimeline was subcutaneously implanted, and then the skin was stitched. Secreted amounts of salivary and tear fluid and sweating were periodically measured from just before the implantation of pump until 32 hours after the administration. The ornithine decarboxylase activity was measured 10 hours after the implantation.
Respective measuring methods are shown below.

a) Measurement of salivary and tear fluid

The mouth of a mouse was wiped with a cotton ball whose weight had been measured in advance, and the incremental weight was used as the amount of salivary fluid. Also at the same time, a thread for tear fluid measurement (Zone Quick; Menicon) used on the Schirmer test was inserted into the lower eyelid conjunctival sac, and the length of colored part was measured and used as the amount of tear fluid. From the measurement results, area under the secreted amount-versus-time curve (AUC) was calculated.

b) Measurement of sweating

The footpad of right hind paw of a mouse anesthetized with diethyl ether was wiped with absorbent cotton, and then 20 μl of each of 5 mg/ml iodine-ethanol solution and 50 mg/ml starch-mineral oil suspension was applied thereto. The number of black spots on footpad was counted and used as the number of activated sweat glands. In the case of the subcutaneous bolus administration test, the number of activated sweat glands 10 minutes after the administration of compound A was measured. In the case of the subcutaneous continuous administration, area under the number of activated sweat glands-versus-time curve (AUC) was calculated.

c) Measurement of ornithine decarboxylase activity in glandular tissue

It is known that ornithine decarboxylase (ODC) activity as a marker of dedifferentiation and growth shows positive correlation with the weight and secretion ability of tissue in the exocrine gland (Nilsson BO et al., 1990, Acta. Physiol. Scand., 140, 105 - 109; Yoshinaga K el al., 1996, Ann. Surg., 224, 139 - 144; Lin CH et al., 1997, J. Pediatr. Gastroenterol. Nutr., 24, 18 - 24; Blume GB et al., 1985, Biochem. Biophys. Res. Commun., 132, 118 - 125). Thus, it can be easily analogized that the ODC activity becomes a useful index of cell growth and function of the exocrine gland. Accordingly, the ability of each drug to activate glandular tissue was evaluated by measuring ODC activity of parotid salivary gland and extra-orbital lacrimal gland.

The amount of $^{14}CO_2$ formed from L-$^{14}$C-ornithine was used as the index of ODC activity.

A mouse anesthetized with diethyl ether was sacrificed by bleeding, and left and right parotid salivary glands and extra-orbital lacrimal glands were isolated. After removing the attached connective tissue and fat, the isolated glandular tissues homogenized in 1.5 ml of a homogenate buffer (25 mM Tris-HCl buffer (pH 7.4) containing 0.1 mM ethylenediaminetetraacetic acid, 0.4 mM pyridoxal phosphate, 5 mM dithiothreitol and 0.1% Brij 35®) using a Potter type homogenizer and then centrifuged at 4°C, 15,000 g for 30 minutes, and the supernatant was used as the enzyme fraction. Reaction of the enzyme and substrate was carried out by adding 100 $\mu$l of a substrate solution (the homogenate buffer containing 300 $\mu$M DL-ornithine and 5 $\mu$Ci L-$^{14}$C-ornithine) to 900 $\mu$l of the enzyme liquid whose protein concentration had been adjusted to 2.5 mg/ml and incubating at 37°C for 1 hour in a sealed tube, and the reaction was terminated by the addition of 100 $\mu$l of 2 M $H_2SO_4$. The $^{14}CO_2$ generated by the reaction was captured by 150 $\mu$l of 1 N NaOH soaked in a glass filter. Radioactivity of the $^{14}CO_2$ captured on the glass filter was detected using a liquid scintillation counter. The enzyme activity (pmol $^{14}CO_2$/mg protein/hour) was calculated from the measured results.

Statistical analysis

All of the test results were expressed as fold increases of the reactions of the drug-administered group versus those of the vehicle-administered group, and comparison between the drug-administered groups and the vehicle-administered group was carried out using Dunnett's t-test. $P < 0.05$ was considered significant.

Results

1) Subcutaneous bolus administration (Example 1, Table 1)

Secretion amount of salivary and tear fluid by the compound A reached maximum in 10 minutes after its administration at every dose. At 10 minutes after the administration, statistically significant acceleration of the secretion of saliva and tear fluid by the compound A was attained at a dose of 10 mg/kg. In addition, statistically significant sweating by the compound A was found at a dose of 30 mg/kg. That is, though the compound A showed the sweating action at a dose 3 times higher than that of accelerating secretion of salivary and tear fluid, it was confirmed that acceleration of the secretion of tear and salivary fluid was attained without accompanying sweating action.

2) Subcutaneous continuous administration of compound A (Example 2, Table 2)

Similar to the case of bolus administration, dose-dependent tear and salivary fluid secretion acceleration action was observed also by the continuous administration of compound A. The secreted amount of tear and salivary fluid started to increase 4 hours after the osmotic pump implantation, became maximum after 10 hours and continued until 26 hours. The secreted amount gradually decreased thereafter, and the secretion acceleration action by the compound A almost disappeared after 32 hours. The secretion acceleration action by the compound A upon both of the lacrimal gland and salivary gland judged by the cumulative tear fluid quantity and salivary fluid quantity showed a statistically significant difference at a dose of 2.5 mg or more in comparison with the vehicle-administered group. Also, this secretion acceleration action reached maximum at 5 mg, and its action strength was maintained even at 10 mg. Further, regardless of the past report stating that the ODC activity acceleration action by a muscarinic receptor agonist requires much higher dose far beyond that for the saliva and tear fluid secretion acceleration action (Kikuchi T et al., 1987, Biochem. Biophys. Res. Com., 44, 1161 - 1166), it was found for the first time that the ODC activity acceleration, namely glandular cell growth action, can also be attained by the continuous administration of compound A at doses which accelerates secretion of tear and salivary fluid without sweating action. In addition, this action was strong, far exceeding the ODC activity acceleration action by the high dose continuous administration of pilocarpine or cevimeline (Comparative Example 1) which is described later.

Sweating acceleration action of the compound A was not found at a dose of up to 10 mg throughout the test period. Accordingly, with the purpose of finding a dose by which the sweating acceleration action is expressed, the sweating action at further higher dose of the compound A was measured. As a result, a statistically significant sweating acceleration action was only confirmed at 20 mg. Thus, it was revealed that the compound A exerts sweating acceleration action at a dose of 8 times higher than the dose which shows statistically significant acceleration actions on tear and salivary fluid secretion and glandular cell growth by a sustained release pharmaceutical preparation form.

3) Subcutaneous bolus administration of pilocarpine or cevimeline (Comparative Example 1, Tables 3 and 4)

Secretion amount of salivary and tear fluid by pilocarpine and cevimeline reached the maximum 10 minutes after the administration.

In this case, statistically significant secretion acceleration of salivary fluid by pilocarpine was attained at a dose of 0.1 mg/kg, and the magnitude of its reaction was not considerably increased even when the dose was increased. On the other hand, a dose of 1 mg/kg which is 10 times higher than the dose for the secretion of salivary fluid was necessary for the secretion of tear fluid. Also, statistically significant sweating acceleration was found at the same dose for accelerating the tear fluid secretion.

Also, statistically significant salivary fluid secretion acceleration action by cevimeline was attained at a dose of 3 mg/kg, but a dose of 10mg/kg which is 3 times higher than the dose for the secretion of salivary fluid was necessary for the secretion acceleration of tear fluid. In addition, similar to the case of pilocarpine, statistically significant acceleration of sweating occurred at the same dose which accelerates tear fluid secretion.

Based on the above, it was confirmed that it is difficult to attain secretion acceleration of both of tear and salivary fluid without accompanying sweating by the bolus administration of pilocarpine or cevimeline.

3) Subcutaneous continuous administration of pilocarpine or cevimeline (Comparative Example 2, Tables 5 and 6)

The saliva secretion acceleration action by the subcutaneous continuous administration of pilocarpine became maximum at 0.16 mg, and further increase was not found even at 0.31 mg. In addition, the ODC activity increasing action of lacrimal gland cell was observed at 0.31 mg which is 2 times higher than the dose for salivary fluid secretion acceleration action. However, the tear fluid secretion acceleration action and the ODC activity increasing action of lacrimal gland cell by the administration of pilocarpine were not able to be found in all of the groups. On the other hand, since distinct sweating acceleration action was found at 0.31 mg, it was revealed that selective and effective stimulation of lacrimal gland and salivary gland cannot be achieved by the continuous administration of pilocarpine.

In the subcutaneous continuous administration of cevimeline, statistically significant salivary fluid secretion acceleration action was observed at 0.63 mg in comparison with the vehicle-administered group, and its action was comparable with the action of the continuous administration of 1.25 mg cevimeline. However, the maximum action of cevimeline on the salivary fluid secretion acceleration was considerably weak in comparison with the compound A, and distinct sweating acceleration action was observed at 1.25 mg which is 2 times higher than the dose for salivary fluid secretion acceleration action, so that similar to the case of pilocarpine, the continuous administration of cevimeline was also not able to stimulate lacrimal gland and salivary gland selectively. In this connection, the glandular cell ODC activity acceleration action of cevimeline was not confirmed even at 1.25 mg which is the sweating acceleration dose.

The above results show that a higher dose than that for salivary fluid secretion acceleration is necessary to attain tear fluid secretion acceleration by the bolus administration of pilocarpine or cevimeline which is effective in improving the dry mouth symptom, and expression of strong side effects cannot be avoided by this dose. On the other hand, by overthrowing the past speculation, it was revealed for the first time that the bolus administration of compound A make it possible to accelerate tear and salivary fluid secretion without accompanying side effects.

In addition, the selective stimulation effect by continuous administration of the compound A upon lacrimal gland and salivary gland is markedly excellent in comparison with that of the continuous administration of cevimeline or pilocarpine, which means that only the component A out of the muscarinic receptor partial agonists can treat dry mouth and dry eye without accompanying side effects, and further can induce regeneration and repair of glandular tissues by accelerating cell growth of damaged lacrimal gland and salivary gland through its sustained release.

Table 1. Reactions of salivary gland, lacrimal gland and sweat gland by subcutaneous bolus administration of compound A

| Evaluation items | Salivary fluid | Tear fluid | Glandular cell growth | | sweating |
|---|---|---|---|---|---|
| | | | Salivary gland | Lacrimal gland | |
| | fold increase vs. the vehicle group | | | | |
| Subcutaneous bolus administration | | | | | |

(continued)

| Evaluation items | Salivary fluid | Tear fluid | Glandular cell growth | | sweating |
|---|---|---|---|---|---|
| | | | Salivary gland | Lacrimal gland | |
| | fold increase vs. the vehicle group | | | | |
| vehicle | 1 | 1 | 1 | 1 | 1 |
| 1 mg/kg | 1.5 ± 0.10 | 1.3 ± 0.11 | | | |
| 3 mg/kg | 1.8 ± 0.13 | 1.4 ± 0.15 | | | 0.69 ± 0.19 |
| 10 mg/kg | 3.3 ± 1.1 (***) | 2.2 ± 0.25 (***) | 0.71 ± 0.05 | 0.99 ± 0.24 | 1.3 ± 0.20 |
| 30 mg/kg | | | 0.72 ± 0.11 | 2.2 ± 0.43 (*) | 2.0 ± 0.19 (**) |
| 100mg/kg | | | 2.8 ± 0.72 (*) | 2.3 ± 0.07 (*) | 2.2 ± 0.17 (***) |
| *: p < 0.05, **: p < 0.01, ***: P < 0.001 (significant difference to vehicle-administered group. Dunnett's test) | | | | | |

A table showing salivary fluid, tear fluid and sweat secretion action and tissue growth action by subcutaneous bolus administration of compound A. The salivary fluid, tear fluid and sweat secretion action shows a result of 10 minutes after the administration of compound A, and the glandular cell growth action shows a result of 6 hours after the administration of compound A. (Reaction acceleration magnitude of the drug-administered group when the solvent-administered group is defined as 1. Average value ± standard deviation)

Table 2. Reactions of salivary gland, lacrimal gland and sweat gland by subcutaneous continuous administration of compound A

| Evaluation items | Salivary fluid | Tear fluid | Glandular cell growth | | sweating |
|---|---|---|---|---|---|
| | | | Salivary gland | Lacrimal gland | |
| | fold increase vs. the vehicle group | | | | |
| Subcutaneous continuous administration | | | | | |
| vehicle | 1 | 1 | 1 | 1 | 1 |
| 0.63 mg | 0.84 ± 0.11 | 1.3 ± 0.11 | | | 0.85 ± 0.06 |
| 1.25 mg | 1.4 ± 0.11 | 1.3 ± 0.011 | 1.56 ± 0.33 | 3.3 ± 1.2 | 0.84 ± 0.06 |
| 2.5 mg | 2.1 ± 0.13 (***) | 1.9 ± 0.10 (***) | 6.9 ± 2.3 (*) | 6.7 ± 0.90 (**) | 0.89 ± 0.05 |
| 5mg | 2.8 ± 0.21 (***) | 2.3 ± 0.08 (***) | 9.6 ± 1.8 (**) | 9.2 ± 1.4 (***) | 0.88 ± 0.05 |
| 10 mg | 3.4 ± 0.35 (***) | 1.6 ± 0.08 (***) | 9.8 ± 2.6 (***) | 7.1 ± 1.2 (***) | 0.90 ± 0.04 |
| 20 mg | | | | | 1.7 ± 0.07 (***) |
| *: p < 0.05, **: p < 0.01, ***: P < 0.001 (significant difference to vehicle-administered group. Dunnett's test) | | | | | |

· A table showing salivary fluid, tear fluid and sweat secretion action and glandular cell growth action by subcutaneous continuous administration of compound A. (Reaction acceleration magnitude of the drug-administered group when the vehicle-administered group is defined as 1. Average value ± standard deviation)
· When the respective doses are expressed by administration rates, they become as follows.
0.025, 0.05, 0.1, 0.2, 0.4 and 0.8 mg/h

Table 3. Reactions of salivary gland, lacrimal gland and sweat gland by subcutaneous bolus administration of pilocarpine

| Evaluation items | Salivary fluid | Tear fluid | Glandular cell growth | | sweating |
|---|---|---|---|---|---|
| | | | Salivary gland | Lacrimal gland | |
| | fold increase vs. the vehicle group | | | | |
| Subcutaneous bolus administration | | | | | |

(continued)

| Evaluation items | Salivary fluid | Tear fluid | Glandular cell growth | | sweating |
|---|---|---|---|---|---|
| | | | Salivary gland | Lacrimal gland | |
| | fold increase vs. the vehicle group | | | | |
| vehicle | 1 | 1 | 1 | 1 | 1 |
| 0.03 mg/kg | 1.2 ± 0.15 | 0.65 ± 0.19 | | | |
| 0.1 mg/kg | 1.8 ± 0.23 (**) | 0.80 ± 0.24 | | | |
| 0.3mg/kg | 1.7 ± 0.15 | 1.1 ± 0.14 | | | 1.1 ± 0.16 |
| 1 mg/kg | 2.0 ± 0.18 (**) | 2.7 ± 0.40 (***) | 1.4 ± 0.48 | 0.69 ± 0.09 | 3.1 ± 0.32 (***) |
| 3 mg/kg | | | 1.3 ± 0.54 | 0.95 ± 0.23 | 4.2 ± 0.29 (***) |
| 10 mg/kg | | | 3.2 ± 1.1 (*) | 2.8 ± 0.61 (**) | |
| *: $p < 0.05$, **: $p < 0.01$, ***: $P < 0.001$ (significant difference to vehicle-administered group. Dunnett's test) | | | | | |

· A table showing salivary fluid, tear fluid and sweat secretion action and tissue growth action by subcutaneous bolus administration of pilocarpine. The salivary fluid, tear fluid and sweat secretion action shows a result of 10 minutes after the administration of compound A, and the glandular cell growth action shows a result of 6 hours after the administration of compound A. (Reaction acceleration magnitude of the drug-administered group when the vehicle-administered group is defined as 1. Average value ± standard deviation)

Table 4. Reactions of salivary gland, lacrimal gland and sweat gland by subcutaneous bolus administration of cevimeline

| Evaluation items | Salivary fluid | Tear fluid | Glandular cell growth | | Sweating |
|---|---|---|---|---|---|
| | | | Salivary gland | Lacrimal gland | |
| | fold increase vs. the vehicle group | | | | |
| Subcutaneous bolus administration | | | | | |
| vehicle | 1 | 1 | 1 | 1 | 1 |
| 0.3 mg/kg | 1.2 ± 0.13 | 1.1 ± 0.14 | | | |
| 1 mg/kg | 2.0 ± 0.22 | 1.1 ± 0.14 | | | |
| 3 mg/kg | 2.1 ± 0.42 (*) | 1.1 ± 0.16 | | | 1.5 ± 0.29 |
| 10 g/k | 3.1 ± 0.50 (***) | 3.6 ± 0.88 (***) | 1.7 ± 065 | 1.2 ± 0.90 | 2.5 ± 0.40 (*) |
| 30 mg/kg | | | 1.1 ± 0.33 | 1.2 ± 0.52 | 3.1 ± 0.57 (**) |
| 100 mg/kg | | | 1.1 ± 0.33 | 1.7 ± 0.79 | |
| *: $p < 0.05$, ** : $p < 0.01$, *** : $P < 0.001$ (significant difference to vehicle-administered group. Dunnett's test) | | | | | |

· A table showing salivary fluid, tear fluid and sweat secretion action and tissue growth action by subcutaneous bolus administration of cevimeline. The salivary fluid, tear fluid and sweat secretion action shows a result of 10 minutes after the administration of compound A, and the glandular cell growth action shows a result of 6 hours after the administration of compound A. (Reaction acceleration magnitude of the drug-administered group when the vehicle-administered group is defined as 1. Average value ± standard deviation)

Table 5. Reactions of salivary gland, lacrimal gland and sweat gland by subcutaneous continuous administration of pilocarpine

| Evaluation items | Salivary fluid | Tearf luid | Glandular cell growth | | Sweating |
|---|---|---|---|---|---|
| | | | Salivary gland | Lacrimal gland | |
| | fold increase vs. vehicle group | | | | |
| Subcutaneous continuous administration | | | | | |
| vehicle | 1 | 1 | 1 | 1 | 1 |
| 0.039 mg | 1.1 ± 0.04 | 0.81 ± 0.02 | | | 1.1 ± 0.10 |
| 0.078mg | 1.5 ± 0.08 | 1.1 ± 0.13 | | | 1.2 ± 0.07 |
| 0.16 mg | 2.9 ± 0.14 (***) | 1.1 ± 0.14 | 0.42 ± 0.06 | 1.4 ± 0.33 | 1.2 ± 0.03 |
| 0.31 mg | 2.8 ± 0.24 (***) | 1.1 ± 0.12 | 1.1 ± 0.20 | 2.0 ± 0.28 (*) | 1.4 ± 0.08 (**) |
| *: $p < 0.05$, **: $p < 0.01$, ***: $P < 0.001$ (significant difference to vehicle-administered group. Dunnett's test) | | | | | |

· A table showing salivary fluid, tear fluid and sweat secretion action and glandular cell growth action by subcutaneous continuous administration of pilocarpine. (Reaction acceleration magnitude of the drug-administered group when the vehicle-administered group is defined as 1. Average value ± standard deviation)
· When the respective doses are expressed by administration rates, they become as follows.
0.00156, 0.00312, 0.0064 and 0.0124 mg/h

Table 6. Reactions of salivary gland, lacrimal gland and sweat gland by subcutaneous continuous administration of cevimeline

| Evaluation items | Salivary fluid | Tear fluid | Glandular cell growth | | sweating |
|---|---|---|---|---|---|
| | | | Salivary gland | Lacrimal gland | |
| | fold increase vs. the solvent group | | | | |
| Subcutaneous continuous administration | | | | | |
| vehicle | 1 | 1 | 1 | 1 | 1 |
| 0.16 mg | 0.98 ± 0.10 | 1.3 ± 0.05 | | | 1.2 ± 0.07 |
| 0.31mg | 1.0 ± 0.12 | 1.1 ± 0.09 | | | 1.2 ± 0.10 |
| 0.63 mg | 1.5 ± 0.11 (*) | 1.4 ± 0.18 | 0.87 ± 0.05 | 1.4 ± 0.28 | 1.4 ± 0.16 |
| 1.25 | 1.7 ± 0.17 (**) | 1.3 ± 0.04 | 0.45 ± 0.12 | 1.3 ± 0.70 | 1.6 ± 0.16 (*) |
| *: $p < 0.05$, **: $p < 0.01$ (significant difference to vehicle-administered group. Dunnett's test) | | | | | |

· A table showing salivary fluid, tear fluid and sweat secretion action and glandular cell growth action by subcutaneous continuous administration of cevimeline. (Reaction acceleration magnitude of the drug-administered group when the vehicle-administered group is defined as 1. Average value ± standard deviation)
· When the respective doses are expressed by administration rates, they become as follows.
0.0064, 0.0124, 0.0252 and 0.05 mg/h

II. Phase I study of compound A (oral administration)

The compound A was orally administered once to healthy male adult volunteers, and saliva secretion action and pharmacokinetics were evaluated.

Method

Drug administration to the volunteers was carried out by a blind method.

In order to observe the volunteers carefully, saliva quantity, sweating and pharmacokinetics (concentrations of unchanged compound A in plasma and urine) were periodically measured.

Results

Acceleration action of salivary fluid secretion was observed starting at a dose of 10 mg and expressed in all cases at 40 mg. On the other hand, sweating was observed in 1 case out of 6 subjects of the 10 mg administration group, and in 5 cases out of 6 subjects of the 60 mg administration group. Table 8 shows pharmacokinetics parameters obtained from this test.

Table 7. Summary of subjective and objective symptoms following compound A administration

| Dose | 0 mg | 5 mg | 10 mg | 20 mg | 40 mg | 60 mg |
|---|---|---|---|---|---|---|
| The number of subjects | 12 | 6 | 6 | 6 | 6 | 6 |
| Increase of saliva secretion | 0 | 0 | 2 | 2 | 6 | 5 |
| Sweating | 0 | 0 | 1 | 0 | 0 | 5 |

Table 8. Pharmakinetic parameters of compound A in plasma following single oral administration

| Dose (mg) | The number of sabjects | Cmax ($\mu$g/ml) | Tmax (hr) | AUC$_{0\rightarrow\infty}$ (ng/hr/ml) | T1/2 (hr) |
|---|---|---|---|---|---|
| 5 | 6 | 19.8 $\pm$ 5.2 | 1.5 $\pm$ 0.55 | 117.6 $\pm$ 41.5 | 3.89 $\pm$ 0.81 |
| 10 | 6 | 35.3 $\pm$ 10.5 | 1.17 $\pm$ 0.41 | 180.5 $\pm$ 77.6 | 3.42 $\pm$ 0.67 |
| 20 | 6 | 68.1 $\pm$ 13.0 | 2.00 $\pm$ 0.63 | 410.1 $\pm$ 106.8 | 3.88 $\pm$ 0.67 |
| 40 | 6 | 151.0 $\pm$ 24.1 | 1.67 $\pm$ 0.82 | 1,010.1 $\pm$ 323.2 | 3.78 $\pm$ 0.59 |
| 60 | 5 | 266.0 $\pm$ 25.2 | 1.60 $\pm$ 0.55 | 1,482.3 $\pm$ 371.1 | 3.20 $\pm$ 0.79 |
| (average value $\pm$ standard deviation) | | | | | |

Example 3 (Hydrogel-forming sustained release pharmaceutical preparation)

A mixed powder comprising the following compositional unit containing the compound A, polyethylene oxide as the hydrogel-forming base and polyethylene glycol as the hydrophilic base was prepared by thoroughly mixing it using a mortar and a pestle until uniformity. A tablet having a weight of 420 mg was prepared by charging the thus prepared mixed powder in dies and subjecting this to compression molding by an oil press tabletting machine using a punch of 9.5 mm diameter x 9.5 R and with a tabletting pressure of 1000 kg/punch.

| | |
|---|---|
| Compound A | 20 mg |
| Polyethylene oxide (Polyox 303; m.w. 7,000,000) | 200 mg |
| Polyethylene glycol (PEG 6000; m.w. 8,000) | 200 mg |
| Total | 420 mg |

Example 4 (Hydrogel-forming sustained release pharmaceutical preparation containing a counter polymer)

A mixed powder comprising the following compositional unit containing the compound A, polyethylene oxide, polyethylene glycol and a carboxy vinyl polymer as the counter polymer having opposite charges to the compound A was prepared by thoroughly mixing it using a mortar and a pestle until uniformity. A tablet having a weight of 420 mg was prepared by charging the thus prepared mixed powder in dies and subjecting this to compression molding by an oil press tabletting machine using a punch of 9.5 mm diameter x 9.5 R and with a tabletting pressure of 1000 kg/punch.

| | |
|---|---|
| Compound A | 20 mg |
| Polyethylene oxide (Polyox 303; m.w. 7,000,000) | 150 mg |
| Carboxy vinyl polymer (Carbopol 971 P) | 50 mg |

(continued)

| Polyethylene glycol (PEG 6000; m.w. 8,000) | 200 mg |
|---|---|
| Total | 420 mg |

Comparative Example 2 (Immediately release pharmaceutical preparation)

A mixed powder consisting of the following compositional unit was prepared by thoroughly mixing a mixed powder consisting of the following compositional unit containing the compound A and lactose as the filler using a mortar and a pestle until uniformity. A tablet having a weight of 420 mg was prepared by charging the thus prepared mixed powder in dies and subjecting this to compression molding by an oil press tabletting machine using a punch of 9.5 mm diameter x 9.5 R and under a tabletting pressure of 1000 kg/punch.

| Compound A | 20 mg |
|---|---|
| Lactose | 400 mg |
| Total | 420 mg |

Test Example 2 (Dissolution test)

Drug release properties from each of the pharmaceutical preparations of Example 3, Example 4 and Comparative Example 2 were evaluated by the dissolution test, second method (paddle method), of The Pharmacopoeia of Japan. The test was carried out using 500 ml of the second fluid of the dissolution test (JP 2 fluid; pH 6.8), without using a sinker, and at a paddle rotating speed of 200 rpm. Samplings were carried out at predetermined periods of time after commencement of the test, and the drug amount in the test fluid was determined using an ultraviolet spectrophotometer. Measuring wavelength of the ultraviolet spectrophotometer was set to 195.4 nm. The thus obtained results are shown in Fig. 1.

(Results and discussion)

Drug release from the Example 3 was considerably delayed in comparison with that of the Comparative Example 2. This is considered to be due to inhibition of disintegration of the pharmaceutical preparation by the formation of hydrogel matrix. Drug release from the Example 4 was further delayed in comparison with that of the Example 3. Since the compound A is a basic drug and further has an amphipathic structure consisting of a hydrophilic moiety and a hydrophobic moiety inside the molecule, it is considered that a cationic molecular micelle is formed in the test liquid. Accordingly, it is considered that, by the addition of an anionic counter polymer (carboxy vinyl polymer) having opposite charges to the compound A, it formed electrostatic interactions with the cations on the micelle surface, thus causing inhibition of diffusion of the drug through the hydrogel matrix and delay of the drug release.

The drug release rate from said pharmaceutical compositions shown in Example 3 and Example 4 can be optionally (e.g., covering from about 2 hours to about 24 hours) controlled, and as described in WO 9406414, US 6436441, US 20030203024, WO 2003/041656 or the like, this can be attained by optionally adjusting blending amount of the hydrogel-forming polymer, blending ratio to the hydrophilic base, blending amount of the counter polymer, and further, a combination of two or more counter polymers and the like.

Based on the above, sustained release of the compound A was shown by the application of a hydrogel matrix or blending of a counter polymer. Thus, it was shown that the compound A according to the invention can be gradually released by these sustained release pharmaceutical preparations.

[Industrial Applicability]

The invention is useful as a result which makes it possible to provide a pharmaceutical composition for the treatment of tear and salivary fluid drying, which accelerates tear and salivary fluid secretion action without accompanying sweating action, and further as a result which makes it possible to provide a pharmaceutical composition for the treatment of tear and salivary fluid drying, which shows lacrimal gland and salivary gland cell growth action without accompanying sweating action, achieved by the sustained drug release.

**Claims**

1. A pharmaceutical composition for the treatment of tear and salivary fluid drying, which comprises (-)-(S)-2,8-dimethyl-3-methylene-1-oxa-8-azaspiro[4.5]decane or a pharmaceutically acceptable salt thereof as the active ingredient.

2. The pharmaceutical composition for the treatment of tear and salivary fluid drying described in claim 1, wherein the active ingredient is L-tartarate monohydrate of the compound described in claim 1.

3. The pharmaceutical composition described in claim 1, which has a selective tear and salivary fluid secretion acceleration action.

4. The pharmaceutical composition described in claim 1 or 3, which has a glandular cell growth action.

5. The pharmaceutical composition described in claim 1 or 3, which has a sustained release preparation form.

6. The pharmaceutical composition described in claim 5, which comprises the compound described in claim 1, or a pharmaceutically acceptable salt thereof, and a sustained release pharmaceutical carrier.

7. The pharmaceutical composition described in claim 2, whose applicable disease is rheumatism, autoimmune diseases, medical diseases, atrophy of salivary gland and lacrimal gland due to aging, allergic keratitis and conjunctivitis, viral diseases, salivary gland and lacrimal gland disorders due to radiation irradiation, aging, psychological fatigue and dryness caused by a side effect at the time of drug administration.

# FIG. 1

## INTERNATIONAL SEARCH REPORT

| | International application No. |
|---|---|
| | PCT/JP2005/018310 |

A.  CLASSIFICATION OF SUBJECT MATTER
*A61K31/438* (2006.01), *A61P1/02* (2006.01), *A61P27/02* (2006.01), *A61P43/00* (2006.01), *C07D491/107* (2006.01)

According to International Patent Classification (IPC) or to both national classification and IPC

B.  FIELDS SEARCHED

Minimum documentation searched (classification system followed by classification symbols)
*A61K31/438* (2006.01), *A61P1/02* (2006.01), *A61P27/02* (2006.01), *A61P43/00* (2006.01), *C07D491/107* (2006.01)

Documentation searched other than minimum documentation to the extent that such documents are included in the fields searched
Jitsuyo Shinan Koho         1922-1996    Jitsuyo Shinan Toroku Koho    1996-2005
Kokai Jitsuyo Shinan Koho   1971-2005    Toroku Jitsuyo Shinan Koho    1994-2005

Electronic data base consulted during the international search (name of data base and, where practicable, search terms used)
REGISTRY(STN), CAplus(STN), BIOSIS(STN), EMBASE(STN), MEDLINE(STN)

C.  DOCUMENTS CONSIDERED TO BE RELEVANT

| Category* | Citation of document, with indication, where appropriate, of the relevant passages | Relevant to claim No. |
|---|---|---|
| Y | JP 2003-063964 A  (Senju Pharmaceutical Co., Ltd.), 05 March, 2003 (05.03.03), Claims; page 3, left column, lines 5 to 13; page 5, left column, lines 28 to 36; examples (Family: none) | 1-7 |
| Y | JP 07-126163 A  (TheraTech Inc.), 16 March, 1995 (16.05.95), Page 4, right column, line 20 to page 5, right column, line 26; examples (Family: none) | 1-3,5-7 |

| ☒ | Further documents are listed in the continuation of Box C. | ☐ | See patent family annex. |
|---|---|---|---|

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 11 November, 2005 (11.11.05) | 22 November, 2005 (22.11.05) |

| Name and mailing address of the ISA/ | Authorized officer |
|---|---|
| Japanese Patent Office | |
| Facsimile No. | Telephone No. |

Form PCT/ISA/210 (second sheet) (April 2005)

<table>
<tr><td colspan="2" align="center">**INTERNATIONAL SEARCH REPORT**</td><td>International application No.<br>PCT/JP2005/018310</td></tr>
</table>

C (Continuation). DOCUMENTS CONSIDERED TO BE RELEVANT

| Category* | Citation of document, with indication, where appropriate, of the relevant passages | Relevant to claim No. |
|---|---|---|
| Y | JP 06-024981 A (Snow Brand Milk Products Co., Ltd.), 01 February, 1994 (01.02.94), Claims; page 2, left column, line 38 to page 4, right column, line 7; examples (Family: none) | 1-4,7 |
| Y | WO 2001/078686 A1 (Yamanouchi Pharmaceutical Co., Ltd.), 25 October, 2001 (25.10.01), Page 7, line 2; page 17, line 8 to page 18, line 13 & US 2002/0028240 A1  & EP 1275381 A1 | 5,6 |
| Y | WO 1994/006414 A1 (Yamanouchi Pharmaceutical Co., Ltd.), 31 March, 1994 (31.03.94), Page 2, line 10 to page 3, line 1; page 5, line 18 to page 8, line 18; examples & JP 2001-010951 A  & US 2003/0203024 A1 & EP 0661045 A1 | 5,6 |
| Y | IWABUCHI, Y. et al., Salivary Secretion and Histopathological Effects after Single Administration of the Muscarinic Agonist SNI-2011 in MRL/Ipr Mice, Archives Internationales de Pharmacodynamie et de Therapie, 1994, Vol.328, No.3, pages 315 to 325 | 4 |

Form PCT/ISA/210 (continuation of second sheet) (April 2005)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 5044948 B
- JP 4020683 A
- JP 2003063964 A
- WO 9406414 A
- WO 0110466 A
- WO 0178686 A
- WO 2003041656 A
- US 6436441 B
- US 6562375 B
- US 20030203024 A
- US 20040091528 A
- US 3845770 A
- US 3916899 A
- US 3995631 A
- US 4008719 A
- US 4111202 A
- US 4160020 A
- US 4327725 A
- US 4519801 A
- US 4578075 A
- US 4681583 A
- US 5019397 A
- US 5156850 A
- US 4077407 A
- US 4088864 A
- US 4994276 A
- US 5128143 A
- US 5135757 A
- US 4839177 A
- US 5422123 A
- US 5780057 A
- US 6149940 A
- US 6340475 B
- US 5972389 A
- US 5582837 A
- US 5007790 A
- WO 9316686 A

**Non-patent literature cited in the description**

- **PORTER SR et al.** An update of the etiology and management of xerostomia. *Oral Surgery Oral Medicine Oral Pathology,* 2004, vol. 97, 28-46
- **CASSOLATO SF et al.** Xerostomia: Clinical Aspects and Treatment. *Gerodontology,* 2003, vol. 20, 64-77
- **GUGGENHEIMER IJ et al.** Xerostomia, Etiology, Recognition and Treatment. *Journal of American Dental Association,* 2003, vol. 134, 61-69
- **SCHAUMBERG DA et al.** Epidemiology of Dry Eye Syndrome. *Advances in Experimental Medicine and Biology,* 2002, vol. 506 (B), 989-998
- **TODA I et al.** Ocular Fatigue is the Major Symptom of Dry Eye. *Acta Ophthalmologica,* 1993, vol. 71, 347-352
- **FOX RI.** Sjogren's Syndrome: Evolving therapies. *Expert Opinion in Investigative Drugs,* 2003, vol. 12, 247-254
- **JOHNSON JT et al.** Oral Pilocarpine for Post-Irradiation Xerostomia in Patients with Head and Neck Cancer. *The New England Journal of Medicine,* 1993, vol. 329, 390-395
- **FOX RI et al.** Update in Sjogren Syndrome. *Current Opinion in Rheumatology,* 2000, vol. 12, 391-398
- **ATKINSON JC et al.** Salivary Enhancement: Current Status and Future Therapies. *Journal of Dental Education,* 2001, vol. 65 (10), 1096-1101
- **ENEROTH CM et al.** Effect of Fractionated Radiotherapy on Salivary Gland Function. *Cancer,* 1972, vol. 30, 1147-1153
- **SHEPPARD JD.** Guidelines for the Treatment of Chronic Dry Eye Disease. *Managed Care,* 2003, vol. 12 (12), 20-25
- **FOX RI et al.** SHORT ANALYTICAL REVIEW, Use of Muscarinic Agonists in the Treatment of Sjogren's Syndrome. *Clinical Immunology,* 2001, vol. 101 (3), 249-263
- **ZIMMERMAN RP et al.** Concomitant Pilocarpine During Head and Neck Irradiation is Associated with Decreased Posttreatment Xerostomia. *International Journal of Radiation Oncology Biology Physics,* 1997, vol. 37 (3), 571-575
- **COPPES RP et al.** Muscarinic Receptor Stimulation Increases Tolerance of Rat Salivary Gland Function to Radiation Damage. *International Journal of Radiation Biology,* 1997, vol. 72 (5), 615-625
- **KIKUCHI K.** Effect of Sialagogues on the Synthesis of Polyamines and DNA in Muraine Parotid Gland. *Biochemical and Biophysical Research Communications,* 1987, vol. 144 (3), 1161-1166

- Osmotic drug delivery: a review of the patent literature. Journal of Controlled Release. 1995, vol. 35, 1-21
- Encyclopedia of Polymer Science and Technology. Interscience Publishers, Inc, 1964, vol. 3, 325-354
- **NILSSON BO et al.** *Acta. Physiol. Scand.,* 1990, vol. 140, 105-109
- **YOSHINAGA K.** *Ann. Surg.,* 1996, vol. 224, 139-144
- **LIN CH et al.** *J. Pediatr. Gastroenterol. Nutr.,* 1997, vol. 24, 18-24
- **BLUME GB et al.** *Biochem. Biophys. Res. Commun.,* 1985, vol. 132, 118-125
- **KIKUCHI T et al.** *Biochem. Biophys. Res. Com.,* 1987, vol. 44, 1161-1166